# EUROPÄISCHE PATENTSCHRIFT

(11) **EP 4 454 696 B1**
(45) Veröffentlichungstag und Bekanntmachung des Hinweises auf die Patenterteilung: **10.12.2025**
(21) Anmeldenummer: 23170017.0
(22) Anmeldetag: 26.04.2023
(51) Int. Cl.: A61M 60/122, A61M 60/178, A61M 60/20, A61M 60/216, A61M 60/50, A61M 60/585, A61M 60/871, A61M 60/88, H01R 13/52

(54) **STEUEREINRICHTUNG FÜR EINE BLUTPUMPE**
CONTROL DEVICE FOR A BLOOD PUMP
DISPOSITIF DE COMMANDE POUR POMPE À SANG

(43) Veröffentlichungstag der Anmeldung: 30.10.2024
(73) Patentinhaber: Berlin Heart GmbH, 12247 Berlin (DE)
(72) Erfinder: Schärff, Eike, 12247 Berlin (DE)
(74) Vertreter: Pfenning, Meinig & Partner mbB

(56) Entgegenhaltungen:
- US-A1- 2014 066 689
- US-A1- 2015 367 047
- US-A1- 2016 022 889
- US-A1- 2021 330 960
- US-B2- 8 596 881

## Beschreibung

Diese Offenbarung betrifft eine Steuereinrichtung für eine Blutpumpe, die eine Steuereinheit und eine Energiespeichereinheit umfasst, die lösbar über eine Steckverbindung elektrisch und mechanisch miteinander verbindbar sind. Ferner betrifft diese Offenbarung eine Blutpumpeneinrichtung, die die beschriebene Steuereinrichtung umfasst.

Im Bereich der Medizintechnik werden erhöhte Anforderungen bzgl. Sicherheit und Bedienbarkeit an Steckverbindungen, die beispielsweise einen elektrischen Akku mit einer Steuereinheit verbinden, gestellt. Verschiedene Realisierungen solcher Steckverbindungen sind bereits aus dem Stand der Technik bekannt.

In EP 2 941 281 A2 wird beispielsweise ein Steuersystem für den Antrieb einer implantierbaren Blutpumpe, umfassend ein erstes Gehäuse mit elektronischen Komponenten, die für den Antrieb der Pumpe konfiguriert sind, und ein zweites Gehäuse, das eine Batterie enthält, beschrieben. Dabei umfassen erstes und zweites Gehäuse mehrere Verriegelungselemente. Ein weiteres Beispiel des Stands der Technik ist aus US 2016/022889 A1 bekannt.

Das Ziel dieser Offenbarung ist es, eine Steuereinrichtung für eine Blutpumpe mit einer verbesserten Steckverbindung bereitzustellen. Dieses Ziel wird durch die Steuereinrichtung für eine Blutpumpe gemäß Anspruch 1 und die Blutpumpeneinrichtung gemäß Anspruch 14 erreicht.

Die Steuereinrichtung umfasst eine Steuereinheit und eine Energiespeichereinheit, die lösbar über eine Steckverbindung elektrisch und mechanisch miteinander verbindbar sind. Dabei weist die Steckverbindung zum Herstellen einer mechanischen Verbindung einen Rahmen und ein in den Rahmen entlang einer Einführungsrichtung einführbares Rahmeneinsteckelement auf, die durch ein Gehäuse der Steuereinheit und ein Gehäuse der Energiespeichereinheit ausgebildet sind. Ferner sind der Rahmen und das Rahmeneinsteckelement so ausgebildet, dass das Rahmeneinsteckelement in zwei oder mehr Orientierungen, die sich durch eine relative Rotation zwischen Rahmen und Rahmeneinsteckelement um die Einführungsrichtung voneinander unterscheiden, in den Rahmen einführbar ist. Zudem weisen der Rahmen und das Rahmeneinsteckelement ein oder mehrere elektrische Kopplungspaare zum Herstellen einer elektrischen Verbindung zwischen Steuereinheit und Energiespeichereinheit auf und die einen oder mehreren elektrischen Kopplungspaare sind so angeordnet, dass eine elektrische Verbindung bei allen Orientierungen möglich ist.

Zur Entwicklung der beschriebenen Steuereinrichtung kam es durch die Einsicht, dass es vorteilhaft sein kann, wenn lösbar verbindbare Einheiten einer Steuereinrichtung mittels einer Steckverbindung, die durch ein Gehäuse der jeweiligen Einheit gebildet wird, vorteilhaft sein können. Dies führt in der Regel dazu, dass die Steuereinrichtung im verbundenen Zustand kompakte Gehäusemaße aufweist. Eine Verbindung der beiden Einheiten mittels bspw. eines Kabels würde nicht zu solch einer kompakten Bauweise führen.

Die beschrieben Steckverbindung der Steuereinrichtung ist typischerweise vorteilhaft, um einen sicheren Wechsel des Energiespeichers zu ermöglichen. Dies wird insbesondere dadurch erreicht, dass eine mechanische wie auch elektrische Verbindung zwischen Steuereinheit und Energiespeicher für alle Orientierungen gegeben ist, die durch Rahmen und Rahmeneinsteckelement von außen durch einen Benutzer erkennbar sind. Dies ist besonders vorteilhaft in Situationen, in denen ein Wechsel des Energiespeichers schnell geschehen muss.

Basierend auf der beschriebenen Steuereinrichtung werden im Folgenden weitere mögliche Ausführungsformen der Steuereinrichtung beschrieben.

Generell kann der Rahmen verschiedene Formen annehmen. In einer Ausführungsform weist der Rahmen eine etwa rechteckige Grundform auf. In einer anderen Ausführungsform weist der Rahmen eine etwa runde, in einer weiteren Ausführungsform eine in etwa quadratische Grundform auf.

Die Energiespeichereinheit weist in den meisten Ausführungsformen der Steuereinrichtung einen Akkumulator zum Speichern von elektrischer Energie auf. In einigen Ausführungsformen weist die Energiespeichereinheit zusätzlich oder alternativ eine Batterie auf.

Zudem weist in den meisten Ausführungsformen die Steuereinrichtung im verbundenen Zustand ein Gesamtgehäuse auf, das zumindest teilweise durch die Gehäuse der Steuereinheit und der Energiespeichereinheit ausgebildet wird.

In einer anderen Ausführungsform weisen das Rahmenelement und das Rahmeneinsteckelement in einer Ebene senkrecht zur Einführungsrichtung zusätzlich oder alternativ eine oder mehrere Spiegelachsen auf. Ferner sind die elektrischen Kopplungspaare symmetrisch bzgl. der einen oder mehreren Spiegelachsen angeordnet. Eine symmetrische Anordnung der elektrischen Kopplungspaare ist typischerweise vorteilhaft, um ein verkannten beim Einführen und Herausziehen des Rahmeneinsteckelementes aus bzw. in den Rahmen zu verhindern. Eine asymmetrische Anordnung der Kopplungspaare ist zwar grundsätzlich auch eine Ausführungsform der Erfindung. Allerdings führt die unterschiedliche Kraftverteilung entlang der Einführungsrichtung beim Herausziehen bzw. Einstecken leichter zu einem Verkannten des Rahmeneinsteckelementes im Rahmen.

In einer weiteren Ausführungsform der Steuereinrichtung weist zusätzlich oder alternativ jedes Kopplungspaar einen an der Steuereinheit angeordneten elektrischen Kontakt und einen an der Energiespeichereinheit angeordneten elektrischen Gegenkontakt auf. In Varianten dieser Ausführungsform sind der elektrische Kontakt und der elektrische Gegenkontakt durch einen Stift bzw. eine Buchse ausgebildet. In einer anderen Variante ist der Stift zusätzlich im Rahmen an der Steuereinheit angeordnet und die Buchse am Rahmeneinsteckelement der Energiespeichereinheit. Diese Variante ist typischerweise besonders sicher, da hier keine mit einer Spannung beaufschlagten Kontakte offen liegen. In einer weiteren Variante ist zusätzlich oder alternativ der Kontakt an einer Rückwand innerhalb des Rahmens angeordnet und/oder der Gegenkontakt an einer Front des Rahmeneinsteckelementes.

In einer weiteren Ausführungsform der Steuereinrichtung ist die Steckverbindung zusätzlich oder alternativ ausgebildet, eine Vielzahl unterschiedlicher elektrischer Größen zwischen Steuereinheit und Energiespeichereinheit zu übertragen und die Steckverbindung für die Übertragung mindestens einer der unterschiedlichen elektrischen Größen zwei elektrische Kopplungspaare umfasst. Dies ist meist vorteilhaft, um eine Redundanz für den Fall eines Ausfalls eines der Kopplungspaare herzustellen und eine Patientensicherheit zu erhöhen. In einer Variante umfasst die Steckverbindung für die Übertragung jeder elektrischen Größe zwei elektrische Kopplungspaare. Als elektrische Größen sind in diesem Zusammenhang sowohl elektrische Signale wie Bus-Signale als auch Massespannung, Phasenspannung etc. zu verstehen.

In einer Ausführungsform der Steuereinrichtung weist die Steckverbindung zusätzlich oder alternativ mindestens zwei elektrische Kopplungspaare auf, wobei die Kopplungspaare so ausgebildet sind, dass beim Einführen des Rahmeneinsteckelementes in den Rahmen eine elektrische Verbindung durch eines der beiden Kopplungspaare hergestellt wird, bevor eine elektrische Verbindung durch ein anderes Kopplungspaar hergestellt wird. Dies ist typischerweise vorteilhaft, um undefinierte Zustände in einer elektrischen Schaltung der Steuereinrichtung zu vermeiden. In einer Variante sind hierfür Elemente des Kopplungspaars, wie Kontakt oder Gegenkontakt, in einer Ebene senkrecht zur Einführungsrichtung vorauseilend angeordnet. In einer anderen Variante ist beispielsweise ein Element des Kopplungspaars für die Masse vorauseilend angeordnet. In einer anderen Variante ist zusätzlich oder alternativ ein Element des Kopplungspaars für die Versorgungsspannung vorauseilend angeordnet.

In einer weiteren Ausführungsform umfasst die Steckverbindung zusätzlich oder alternativ einen lösbaren, mechanischen Verschluss, der ausgebildet ist, nach dem Einführen des Rahmeneinsteckelementes in den Rahmen unabhängig von der Orientierung ein Entfernen des Rahmeneinsteckelementes aus dem Rahmen zu verhindern. In einer Variante umfasst der lösbare mechanische Verschluss zum Verriegeln und Lösen einen eine Rastnase aufweisenden Rasthaken, eine Rastöffnung und ein erstes Betätigungselement, wobei in einer Ruheposition der Rasthaken in die Rastöffnung derart eingreift, dass der Rasthaken durch die Rastöffnung hindurchragt und die Rastnase eine Umrandung der Rastöffnung zumindest teilweise hintergreift, in einer Vorlöseposition der Rasthaken zumindest teilweise in die Rastöffnung derart eingreift, dass zumindest ein Teil des Rasthakens durch die Rastöffnung hindurchragt und die Rastnase von der Umrandung der Rastöffnung gelöst ist, in einer Freigabeposition der Rasthaken außerhalb der Rastöffnung angeordnet ist, wobei das Betätigungselement derart ausgebildet ist, dass eine Betätigung des Betätigungselements den Rasthaken und/oder die Rastöffnung von der Vorlöseposition in die Freigabeposition bewegt. In einer anderen Variante sind zusätzlich oder alternativ Elemente des lösbaren mechanischen Verschlusses symmetrisch zu der einen oder den mehreren Spiegelachsen des Rahmen bzw. des Rahmeneinsteckelementes angeordnet.

In einer anderen Ausführungsform umfasst zusätzlich oder alternativ ein elektrisches Kopplungspaar als elektrischen Kontakt eine Buchse und als elektrischen Gegenkontakt einen in die Buchse einführbaren Stift, wobei der Stift in Einführungsrichtung längenveränderbar ist und eine Feder umfasst, die ausgebildet ist, mittels einer Rückstellkraft einem Zusammendrücken des Stiftes entgegenzuwirken, und Buchse und Stift so angeordnet sind, dass im verbundenen Zustand der Steckverbindung der Stift durch die Buchse zusammengedrückt wird. Diese Ausführungsform des Kopplungspaares ist typischerweise vorteilhaft, um eine sichere elektrische Verbindung auch bei bspw. Erschütterungen und Herstellungstoleranzen der Kopplungspaare zu ermöglichen.

In einer Variante der Steuereinrichtung, die den lösbaren mechanischen Verschluss und das elektrische Kopplungspaar mit Feder umfasst, ist die in dem elektrischen Kopplungspaar verwendete Feder so ausgebildet, dass die Feder eine Rückstellkraft ausbilden kann, die größer ist als eine zum Verschließen des mechanischen Verschlusses benötigte Kraft. Dies hat den typischerweise den Vorteil, dass das Rahmeneinsteckelement durch die Feder wieder aus Rahmen herausgedrückt, wenn Verschluss nicht greift. In einer anderen Variante umfassen mehrere Kopplungspaare eine Feder und eine Summe der Rückstellkräfte aller Federn ist größer als eine zum Verschließen des mechanischen Verschlusses benötigte Kraft.

In einer weiteren Ausführungsform der Steuereinrichtung umfasst zusätzlich oder alternativ die Steckverbindung ein Dichtungselement, das so angeordnet ist, dass in einem verbundenen Zustand der Steckverbindung ein In-Kontakt-kommen von Staub und/oder Wasser mit dem einen oder den mehreren elektrischen Kopplungspaaren reduziert wird. In einer Variante wird durch die Dichtung ein In-Kontakt-Kommen weitestgehend verhindert.

In einer noch anderen Ausführungsform umfasst zusätzlich oder alternativ ein elektrisches Kopplungspaar als elektrischen Kontakt eine Stabelektrode und als elektrischen Gegenkontakt eine Stabbuchse, wobei die Stabelektrode entlang einer axialen Richtung der Stabelektrode eine Vielzahl von Stabkontaktelementen zur unabhängigen Übertragung von zwei oder mehr elektrischen Größen aufweist, und die Stabbuchse entsprechend der Stabelektrode entlang einer axialen Richtung der Stabbuchse eine Vielzahl von Buchsenkontaktelementen aufweist. In einer Variante sind die Buchsenkontaktelemente als Ringfederkontakte ausgebildet und/oder die Stabkontaktelemente als zylindrische Kontaktflächen.

In einer weiteren Ausführungsform weist ein elektronisches Kopplungspaar zusätzlich oder alternativ einen elektrischen Abnehmer und eine elektrische Kontaktfläche auf, wobei die elektrische Kontaktfläche an einer senkrecht zur Einführungsrichtung zeigenden Seite des Rahmeneinsteckelementes angeordnet ist, und der elektrische Abnehmer entsprechend zur elektrischen Kontaktfläche an einer Innenseite des Rahmens angeordnet ist.

In einer anderen Ausführungsform weisen der Rahmen und das Rahmeneinsteckelement in einer Ebene senkrecht zur Einführungsrichtung zusätzlich oder alternativ eine Grundform auf, die so ausgebildet ist, dass das Rahmeneinsteckelement in zwei Orientierungen, die sich durch eine relative Rotation zwischen Rahmen und Rahmeneinsteckelement von 180° um eine Einführungsrichtung voneinander unterscheiden, in den Rahmen einführbar ist.

In einer noch anderen Ausführungsform sind Kontakte der Kopplungspaare des Rahmens und des Rahmeneinsteckelementes zusätzlich oder alternativ cluster-artig innerhalb des Rahmens bzw. am Rahmeneinsteckelement angeordnet, wobei ein Abstand zwischen Kontakten eines Clusters kleiner ist als ein Abstand zwischen den Clustern.

In einer weiteren Ausführungsform umfasst die Steckverbindung zusätzlich oder alternativ ein Führungselement und eine geführtes Element, die so an Rahmen und Rahmeneinsteckelement angeordnet sind, dass während eines Einführens des Rahmeneinsteckelementes in den Rahmen, das Führungselement das geführte Element entlang der Einführungsrichtung führt. Im Folgenden wird nun die Blutpumpeneinrichtung gemäß dieser Offenbarung beschrieben. Die Blutpumpeneinrichtung umfasst eine implantierbare Blutpumpe und eine Steuereinrichtung gemäß einer der vorhergehend beschriebenen Ausführungsformen, wobei die Steuereinrichtung elektrisch mit der Blutpumpe verbunden ist. Die Blutpumpeneinrichtung hat typischerweise die gleichen Vorteile wie die zuvor beschriebene Steuereinrichtung.

In einer Ausführungsform der Blutpumpeneinrichtung ist die elektrische Verbindung zwischen Blutpumpe und Steuereinrichtung kabelgebunden realisiert. In einer anderen Ausführungsform ist die elektrische Verbindung zusätzlich oder alternativ kabellos (bspw. durch Induktion) realisiert. In weiteren Ausführungsformen dient die Verbindung zusätzlich oder alternativ zur Steuerung und/oder zur Versorgung der Blutpumpe mit elektrischer Energie.

Im Folgenden werden nun Ausführungsbeispiele der Steuereinrichtung wie auch der Blutpumpeneinrichtung anhand der Figuren erläutert. Zunächst wird dazu eine Übersicht der Figuren gegeben.
- Fig. 1: zeigt ein Ausführungsbeispiel einer Steuereinrichtung für eine Blutpumpe, die eine Steuereinheit und eine Energiespeichereinheit umfasst;
- Fig. 2A: zeigt eine Frontalansicht der Steuereinheit aus Fig. 1 mit Blick entlang einer Einführungsrichtung;
- Fig. 2B: zeigt eine Frontalansicht der Energiespeichereinheit aus Fig. 1 mit Blick entgegengesetzt zur Einführungsrichtung;
- Fig. 3A: zeigt eine Frontalansicht einer Anordnung von Kontakten der Steuereinrichtung aus Fig. 1;
- Fign. 3B-E: zeigen zu Fig. 3A alternative Anordnungen von Kontakten;
- Fig. 4A: zeigt einen Querschnitt durch die Steuereinrichtung aus Fig. 1 in einem verbundenen Zustand entlang der Einführungsrichtung;
- Fig. 4B: zeigt eine Steckverbindung der Steuereinrichtung aus Fig. 1 in getrenntem Zustand;
- Fig. 5: zeigt eine Steuereinrichtung mit zwei Stabelektroden und zwei Stabbuchsen zum Herstellen eines elektrischen Kontakts;
- Fig. 6: zeigt eine Vergrößerung der Stabelektroden und Stabbuchsen der Steuereinrichtung aus Fig. 5 im verbunden Zustand;
- Fig. 7A: zeigt eine Frontalansicht einer alternativen Steuereinheit mit nur einer Stabelektrode;
- Fig. 7B: zeigt eine Frontalansicht einer zu in Fig. 7A gezeigten Steuereinheit entsprechenden Energiespeichereinheit mit nur einer Stabbuchse;
- Fig. 8: zeigt eine Energiespeichereinheit, mit einer cluster-artigen Anordnung von Buchsen; und
- Fig. 9: zeigt eine Blutpumpeneinrichtung.

Im Folgenden folgt nun eine detaillierte Beschreibung der in den Figuren gezeigten Ausführungsbeispiele.

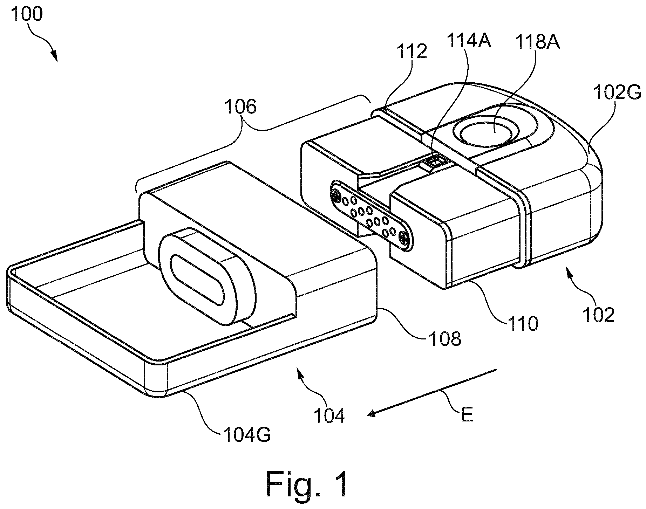

Ein erstes Ausführungsbeispiel einer Steuereinrichtung wird dabei zunächst mit Bezug auf die Fign. 1-4 beschrieben.

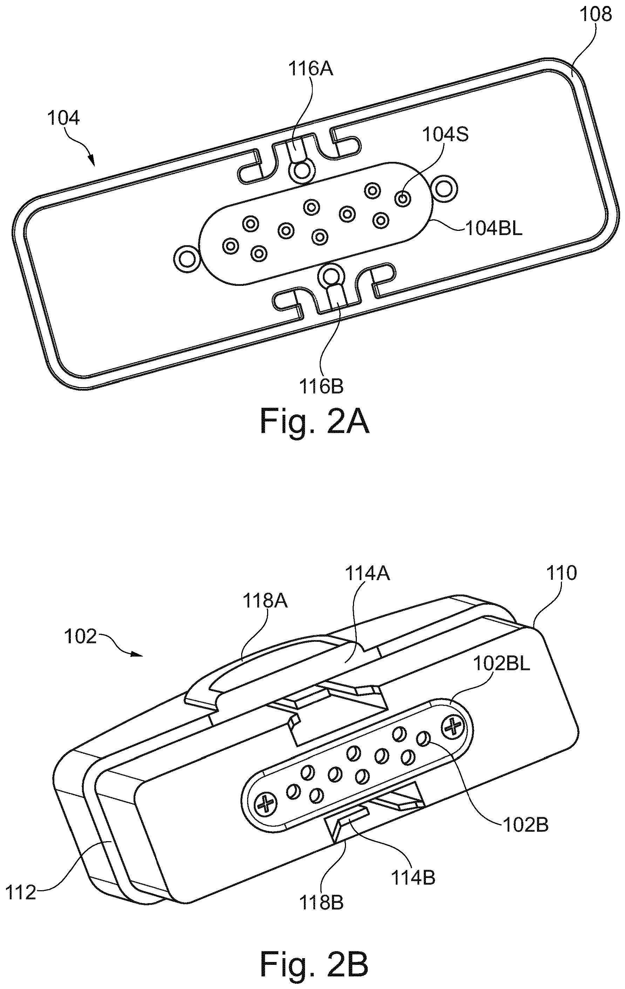

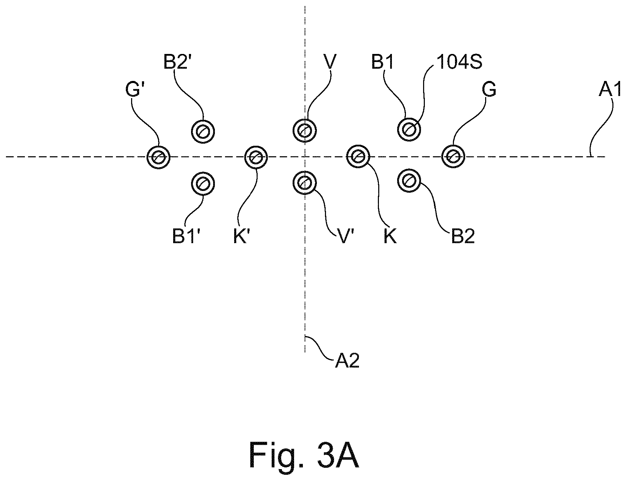

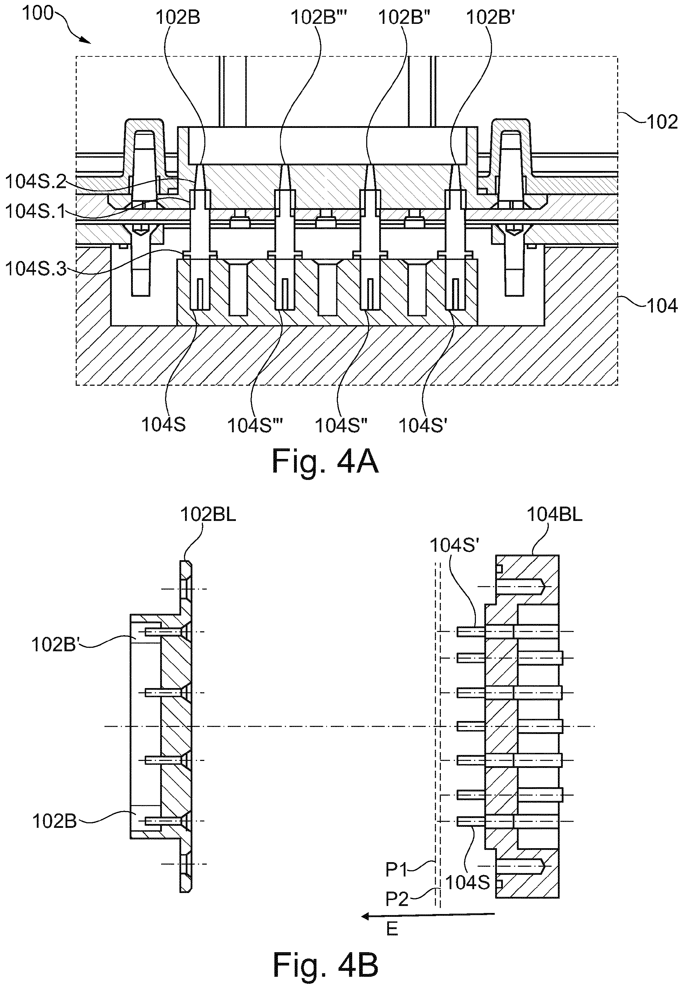

Fig. 1 zeigt ein Ausführungsbeispiel einer Steuereinrichtung 100 für eine Blutpumpe, die eine Steuereinheit 104 und eine Energiespeichereinheit 102 umfasst. Fig. 2A zeigt eine Frontalansicht der Steuereinheit 104 aus Fig. 1 mit Blick entlang der Einführungsrichtung E. Fig. 2B zeigt eine Frontalansicht der Energiespeichereinheit 102 aus Fig. 1 mit Blick entgegengesetzt zur Einführungsrichtung E. Fig. 3A zeigt eine Frontalansicht einer Vielzahl Kontakte der Steuereinheit 104 aus Fig. 1. Fig. 4A zeigt einen Querschnitt durch die Steuereinrichtung 100 aus Fig. 1 in einem verbundenen Zustand entlang der Einführungsrichtung E. Identische Bestandteile der in den Fign. 1-4 gezeigten Steuereinrichtung 100 sind in allen Figuren mit identischen Bezugszeichen versehen.

Die Steuereinheit 104 und die Energiespeichereinheit 102 sind lösbar über eine Steckverbindung 106 elektrisch und mechanisch miteinander verbindbar. Die Steckverbindung 106 umfasst zum Herstellen einer mechanischen Verbindung einen Rahmen 108 und ein in den Rahmen entlang einer Einführungsrichtung E einführbares Rahmeneinsteckelement 110 aufweist, die durch ein Gehäuse 104G der Steuereinheit 104 und ein Gehäuse 102G der Energiespeichereinheit 102 ausgebildet sind. Weiterhin sind der Rahmen 108 und das Rahmeneinsteckelement 110 so ausgebildet, dass das Rahmeneinsteckelement 110 in zwei oder mehr Orientierungen, die sich durch eine relative Rotation zwischen Rahmen 108 und Rahmeneinsteckelement 110 um die Einführungsrichtung E voneinander unterscheiden, in den Rahmen 108 einführbar ist. Zudem weisen der Rahmen 108 und das Rahmeneinsteckelement 110 mehrere elektrische Kopplungspaare zum Herstellen einer elektrischen Verbindung zwischen Steuereinheit 104 und Energiespeichereinheit 102 auf und die mehreren elektrischen Kopplungspaare sind so angeordnet, dass eine elektrische Verbindung zwischen Energiespeichereinheit 102 und Steuereinheit 104 bei allen Orientierungen möglich ist.

In der Darstellung in Fig. 1 ist zur Vereinfachung nur ein Teil des Gehäuses 104G dargestellt, um eine Rückseite eines Teils der elektrischen Kopplungspaare, die an der Steuereinheit 104 angeordnet sind, sichtbar zu machen. Das Gehäuse 102G und das Gehäuse 104G sind in der gezeigten Ausführungsform aus einem thermoplastischen Kunststoff hergestellt. Mögliche Werkstoffe für die Herstellung des Kunststoffes sind ABS-PC, ABS, Polyurethan und PMMA.

Der Rahmen 108 und das Rahmeneinsteckelement 110 weisen eine Rechteckige Grundform auf und besitzen daher zwei Orientierungen, die ein Einstecken des Rahmeneinsteckelementes 110 in den Rahmen 108 ermöglichen - eine erste Orientierung und eine zweite Orientierung, bei der Steuereinheit 104 und Energiespeichereinheit 102 um 180° um die Einführungsrichtung E relativ zueinander gedreht werden.

Das in Fign. 1-4 gezeigte Ausführungsbeispiel der Steuereinrichtung 100 umfasst insgesamt 10 Kopplungspaare, wobei jedes Kopplungspaar ein an der Steuereinheit 104 angeordneten Stift 104S und einer an der Energiespeichereinheit 102 angeordnete Buchse 102B aufweist. Die Anordnung des Stiftes 104S an der Steuereinheit 104 und der Buchse 102B an der Energiespeichereinheit 102 ist nicht zwingend erforderlich, d.h. der Stift könnte auch an der Energiespeichereinheit und die Buchse an der Steuereinheit angeordnet sein. Für die Sicherheit eines Benutzers wie auch für den Schutz der Einrichtung ist die gezeigte Anordnung aber vorteilhaft, da ein Kontakt an der Energiespeichereinheit 102 in einem Inneren der Buchse 102B liegt und so nicht direkt zugänglich ist. Die Stifte der Steuereinheit 104 sind so ausgebildet, dass sie in die Buchsen der Energiespeichereinheit 102 eingeführt werden können, um eine elektrisch leitende Verbindung zwischen der Steuereinheit 104 und der Energiespeichereinheit 102 herzustellen. Ferner sind die Stifte an einer Rückwand innerhalb des Rahmens 108 angeordnet und die Buchsen an einer Front des Rahmeneinsteckelementes 110. Die Anzahl der Kopplungspaare kann beliebig skaliert werden und kann damit an die für die jeweilige Anwendung benötigte Anzahl an elektrischen Kontakten angepasst werden.

In Fig. 3A ist noch einmal eine vergrößerte Ansicht der Stifte dargestellt, wie sie innerhalb des Rahmens 108 der Steuereinheit 104 angeordnet sind. Wie in Fig. 3A zu sehen ist, sind die Stifte symmetrisch zu Spiegelachse A1 und Spiegelachse A2 angeordnet. Die Spiegelachsen A1 und A2 entsprechen dabei den Spiegelachsen des Rahmens 108 in einer Ebene senkrecht zur Einführungsrichtung E. Weiterhin ist in Fig. 3A die Belegung der Kopplungspaare mit den Bezugszeichen B1, B2, G, K und V angedeutet. Dabei dienen die Kontakte B1 und B2 für eine Buskommunikation, der Kontakt G für eine Masseverbindung, der Kontakt V für eine Spannungsverbindung und der Kontakt K zur Feststellung einen Kontaktes zwischen Steuereinheit 104 und Energiespeichereinheit 102. Jeder Kontakt (und jeder Gegenkontakt an der Speichereinheit 102) ist dabei redundant ausgelegt und zweimal vorhanden, was mit den gestrichenen Bezugszeichen B1', B2', G' und K' verdeutlicht werden soll. Weiterhin sind die redundanten Kopplungspaare so angeordnet, dass eine elektrische Verbindung entsprechend jedes Kontaktes in allen Orientierungen von Rahmen 108 zu Rahmeneinsteckelement 110 möglich ist.

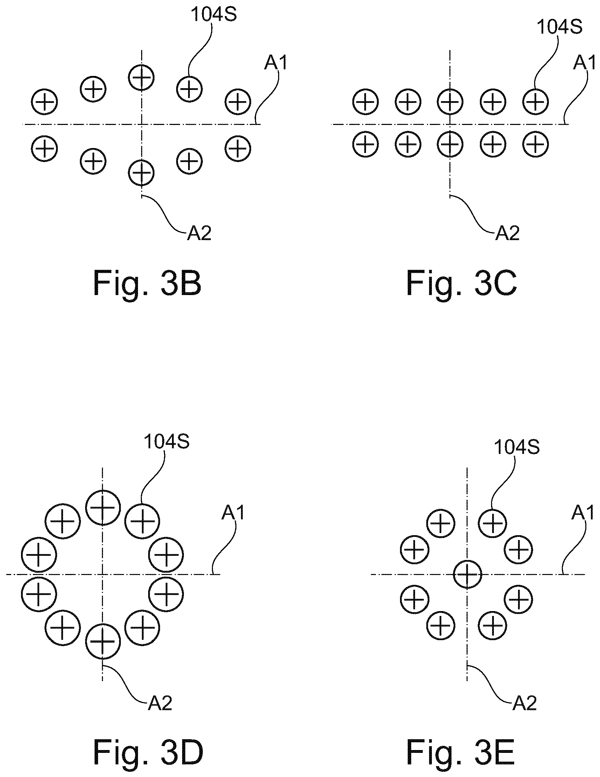

In Fig. 3A ist nur eine mögliche Anordnung von Stiften 104S gezeigt. Auch andere Anordnungen sind möglich. Fig. 3B-E zeigen alternative Anordnungen der Kontakte. Die in den Figuren gezeigten Anordnungen sind alle symmetrisch zu den Achsen A1 und A2. In Fig. 3B und 3C sind die Kontakte dabei in zwei Reihen jeweils oberhalb und unterhalb der Achse A1 angeordnet. Während in Fig. 3C die Stifte 104S in einer Reihe parallel zur Achse A1 angeordnet sind, verlaufen in Fig. 3B die Reihen ober und unterhalb der Achse A1 leicht bogenförmig. In Fig. 3D und Fig. 3E sind die Kontakte kreisförmig um einen Schnittpunkt zwischen der Achse A1 und der Achse A2 angeordnet. Die in Fig. 3E gezeigte Anordnung umfasst dabei zusätzlich einen Kontakt, der auf dem Schnittpunkt zwischen Achse A1 und Achse A2 angeordnet ist. Dies ermöglicht eine ungerade Anzahl an Kontakten bei gleichzeitig symmetrischer Anordnung bzgl. der Achse A1 und der Achse A2. Weitere Anordnungen werden später mit Bezug auf andere Ausführungsbeispiele beschrieben.

Die Steuereinrichtung 100 umfasst weiterhin einen lösbaren mechanischen Verschluss, der ausgebildet ist, nach dem Einführen des Rahmeneinsteckelementes 110 in den Rahmen 108 unabhängig von der Orientierung ein Entfernen des Rahmeneinsteckelementes 110 aus dem Rahmen 108 zu verhindern. Im Ausführungsbeispiel der Fign. 1-4 umfasst der mechanische Verschluss zwei auf gegenüberliegenden Seiten des Rahmeneinsteckelementes 110 angeordnete Rastnasen 114A und 114B, die ausgebildet sind, in einem verbundenen Zustand ein Rahmenelement 116A bzw. 116B einer Rastöffnung zu hintergreifen. Zwei auf sich gegenüberliegenden Seiten der Energiespeichereinheit 102 angeordnete Betätigungselemente 118A und 118B sind ausgebildet, bei einem Betätigen, die mechanische Verbindung zwischen Rastnase und jeweiligem Rahmenelement zu lösen. Im Bereich der Rastnasen 114A und 114B gibt es zusätzlich eine Einkerbung, die sich in Einführungsrichtung erstreckt. Diese Einkerbungen können jeweils als Führungselement dienen, um die Rahmenelemente 116A und 116B beim Einführen von Rahmeneinsteckelement 110 in den Rahmen 108 zu führen.

Zudem umfasst die Steckverbindung 106 ein Dichtungselement 112, das um das Rahmeneinsteckelement 110 herumläuft und in einem verbundenen Zustand der Steckverbindung 106 mit dem Rahmen 108 zusammenwirkt, um ein In-Kontakt-kommen von beispielsweise Staub und Wasser mit den elektrischen Kopplungspaaren zu reduzieren.

In Fig. 4A ist ein Querschnitt durch die Steuereinrichtung 100 entlang der Einführungsrichtung E in einem verbundenen Zustand gezeigt. Abgebildet ist, wie in dem verbundene Zustand die Stifte der Steuereinheit 104 in die Buchsen der Energiespeichereinheit 102 eingeführt sind, um eine elektrisch leitende Verbindung herzustellen. Weiterhin sind im Querschnitt noch weitere Details zum Aufbau der Stift gezeigt. Exemplarisch wird dieser Aufbau für den Stift 104S beschrieben. Der Stift 104S ist in Einführungsrichtung längenveränderbar und umfasst einen Stiftfuß 1045.3, der eine Feder umfasst (nicht gezeigt), die ausgebildet ist, mittels einer Rückstellkraft einem Zusammendrücken des Stiftes 104S entgegenzuwirken. Die Längenveränderbarkeit des Stiftes 104S wird in dem gezeigten Beispiel durch einen teleskopstabartigen Aufbau des Stiftes 104S erreicht. Dazu umfasst der Stift 104S eine Hülse 104S.1, die entlang der Einführungsrichtung E axial ausgerichtet ist und eine in der Hülse 104S.1 axial bewegbar angeordnete Spitze 104S.2. Die Spitze 1045.2 wird dabei durch die Feder im Stiftfuß 104S.3 in einem von der Steuereinheit 110 abgewandtes Ende der Hülse 104S.1 gedrückt. Weiterhin sind Buchse 102B und Stift 104S so angeordnet, dass im verbundenen Zustand der Steckverbindung 106 der Stift 104S durch die Buchse 102B zusammengedrückt wird, um auch bei Produktionstoleranzen eine elektrisch leitende Verbindung zu ermöglichen. Zudem sind die in den elektrischen Kopplungspaaren verwendeten Federn so ausgebildet, dass die Federn in Summe eine Rückstellkraft ausbilden, die größer ist als eine zum Verschließen des mechanischen Verschlusses benötigte Kraft. Hierdurch wird das Rahmeneinsteckelement 110 aus dem Rahmen 108 herausgedrückt, wenn der mechanische Verschluss noch nicht richtig eingerastet ist. Dies ist typischerweise vorteilhaft, damit ein Benutzer einen Fehler beim Verbinden von Rahmen 108 und Rahmeneinsteckelement 110 sofort erkennen kann.

Fig. 4B zeigt die Steckverbindung aus Fig. 4A in getrenntem Zustand. Insbesondere ist in Fig. 4B das Blendenelement 102BL mit den Buchsen sowie das Blendenelement 104BL mit den Stiften gezeigt. In dieser Abbildung ist zu erkennen, wie Stifte 104S, 104S'so angeordnet sind, dass beim Einführen des Rahmeneinsteckelementes 110 in den Rahmen 108 eine elektrische Verbindung durch eines der beiden Kopplungspaare hergestellt wird, bevor eine elektrische Verbindung durch die anderen Stifte hergestellt wird, und beim Herausnehmen des Rahmeneinsteckelementes 110 aus dem Rahmen 108 die elektrische Verbindung der Stifte 104S und 104S' als letztes getrennt wird. Dies wird im Ausführungsbeispiel 100 erreicht, in dem die Stifte 104S, 104S'in Einführungsrichtung E vorauseilend gegenüber den anderen Stiften angeordnet sind, wie anhand der beiden in Fig. 4B angedeuteten Ebenen P1 und P2 deutlich wird. In anderen, hier nicht gezeigten Ausführungsformen sind alternativ die Buchsen 102B und 102B' vorauseilend angeordnet, wodurch der gleiche Effekt erreicht wird.

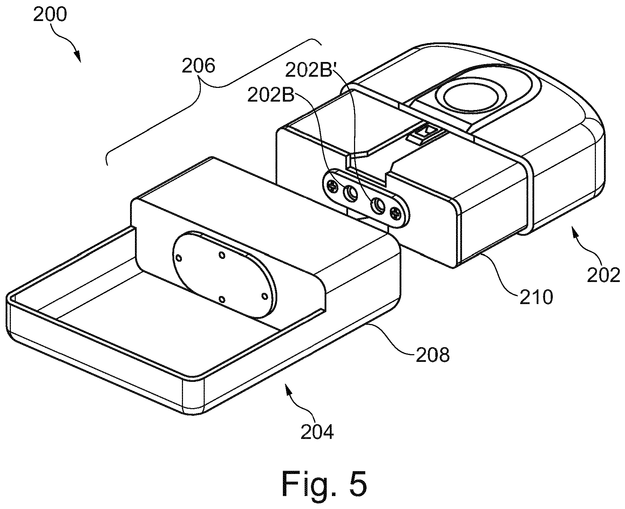

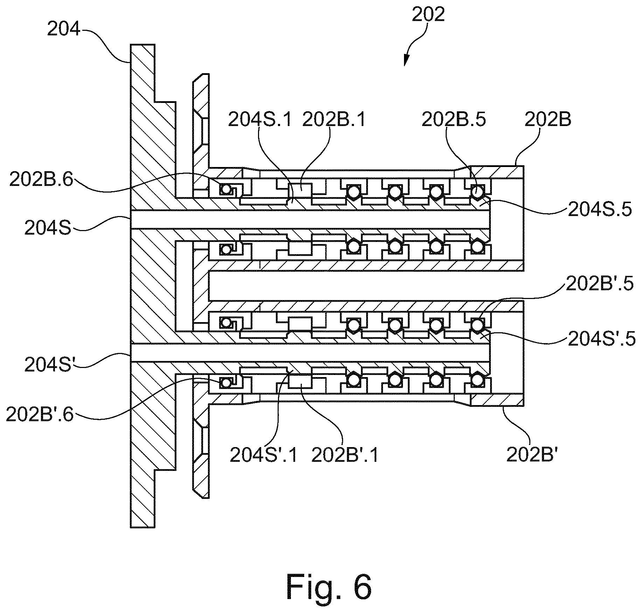

Im Folgenden wird nun noch ein weiteres Ausführungsbeispiel einer Steuereinrichtung mit Bezug auf Fig. 5 und Fig. 6 beschrieben.

Fig. 5 zeigt eine Steuereinrichtung 200 mit zwei Stabelektroden 204S, 204S'und zwei Stabbuchsen 202B, 202B' zum Herstellen eines elektrischen Kontakts. Fig. 6 zeigt eine Vergrößerung der Stabelektroden 204S, 204S' und Stabbuchsen 202B, 202B' der Steuereinrichtung 200 aus Fig. 5 im verbundenen Zustand.

Die Steuereinrichtung 200 ist weitestgehend identisch zur Steuereinrichtung 100. Die Steuereinrichtung 200 umfasst eine Energiespeichereinheit 202 und eine Steuereinheit 204, die über eine Steckverbindung 206 mechanisch und elektrisch lösbar miteinander verbunden sind. Von der Steckverbindung 206 ist dazu ein an der Steuereinheit 204 angeordneter Rahmen 208 umfasst und ein an der Energiespeichereinheit 204 angeordnete Rahmeneinsteckeinheit 210. Die Steuereinrichtung 200 unterscheidet sich von der Steuereinrichtung 100 in erster Linie in Bezug auf die Herstellung einer elektrischen Verbindung zwischen Steuereinheit 204 und Energiespeichereinheit 202. Hierzu umfasst die Steuereinheit 204 die zwei im Rahmen 208 angeordnete Stabelektroden 204S und 204S' und die zwei am Rahmeneinsteckelement 210 angeordneten Stabbuchsen 202B, 202B'. Wie in Fig. 6 gezeigt ist, umfassen die Stabelektroden 202S, 202S' fünf entlang einer Längsachse der Stabelektroden axial hintereinander angeordnete zylindrische Stabkontaktelemente 204S.1-5 bzw. 204S'.1-5. Jede der Stabkontaktelemente ist dabei so angeordnet, dass sie einen elektrisch leitenden Kontakt mit einem von jeweils fünf in Längsrichtung axial angeordneten Buchsenkontaktelementen 202B.1-5, 202B'.1-5 herstellen können. Die Buchsenkontaktelemente 202B.1-5, 202B'.1-5 sind dabei als Ringfederkontakte ausgebildet. Nebeneinander liegende Ringfederkontakte sind durch dazwischenliegende Isolatoren voneinander elektrisch isoliert. Die Ringfederkontakte 202B.1-5, 202B'.1-5 liegen in einem Gehäuse aus einem dielektrischen Material wie beispielsweise Kunststoff oder Keramik. Weiterhin umfasst jeder der Stabbuchsen 202B, 202B' eine Dichtung 202B.6, 202B'.6, um ein Eindringen von Staub und Flüssigkeiten in die Buchsen im verbundenen Zustand zu verhindern. Die elektrischen Kontakte der Stabelektroden 204S, 204S' und der Stabbuchsen 202B, 202B' sind jeweils so ausgelegt, dass auch bei einer Rotation von Steuereinheit 204 und Energiespeicherelement um 180° um die Einführungsrichtung E relativ zueinander eine entsprechende richtige Kontaktierung hergestellt wird.

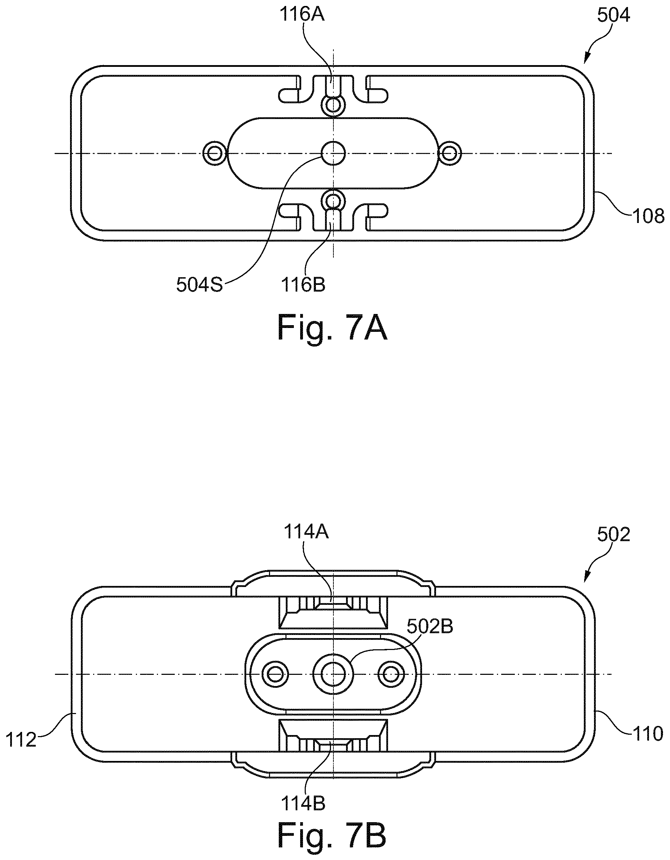

In dem Ausführungsbeispiel 200 der Steuereinrichtung werden zwei Stabelektroden bzw. Stabbuchsen verwendet, um eine Redundanz herzustellen. Alternativ hierzu ist es auch möglich, nur eine Stabelektrode zu verwenden. Solch ein Ausführungsbeispiel einer Steuereinrichtung ist in Fig. 7A und Fig. 7B gezeigt.

Fig. 7A zeigt eine Frontalansicht einer alternativen Steuereinheit 504 mit nur einer Stabelektrode 504S. Fig. 7B zeigt eine Frontalansicht einer zu in Fig. 7A gezeigten Steuereinheit 504 entsprechenden Energiespeichereinheit 502 mit nur einer Stabbuchse 502B. Die Stabelektrode 504S und die Stabbuchse 502B sind genauso aufgebaut wie die zuvor besprochenen Stabelektroden 204S, 204S' bzw. wie die Stabbuchsen 202B, 202B'. Durch eine Anordnung der Stabelektrode 504S bzw. Stabbuchse 502B mittig innerhalb des Rahmens 108 bzw. des Rahmeneinsteckelementes 110 ist es auch hier möglich, das Rahmeneinsteckelement bei einer Drehung um 180° bzgl. des Rahmenelementes 108 um die Einsteckrichtung E miteinander elektrisch und mechanisch in Kontakt zu bringen.

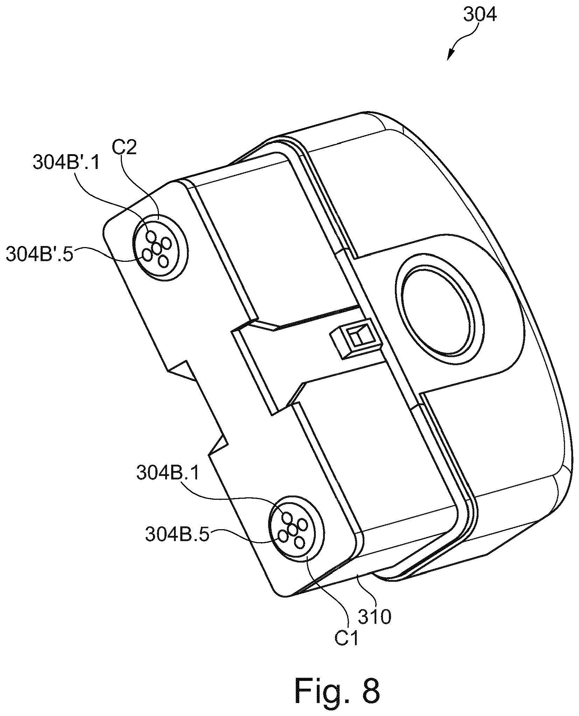

Weitere Ausführungsbeispiele weisen zudem auch eine andere Anordnung von Kontakten und Gegenkontakten an Rahmen und Rahmeneinsteckelement auf. Im Folgenden soll nun mit Bezug auf Fig. 8 ein weiteres Ausführungsbeispiel beschrieben werden.

Fig. 8 zeigt eine Energiespeichereinheit 304, mit einer cluster-artigen Anordnung von Buchsen.

Die Energiespeichereinheit 304 umfasst analog wie andere bereits besprochene Ausführungsbeispiele eine Rahmeneinsteckeinheit 310. An der Rahmeneinsteckeinheit 310 sind jeweils Buchsen 304B.1-5,304B'.1-5 in zwei Clustern C1 und C2 angeordnet. Die Cluster C1 und C2 definieren sich dadurch, dass ein Abstand zwischen Kontakten eines Clusters kleiner ist als ein Abstand zwischen den Clustern. Die Buchsen selbst sind auch bei diesem Ausführungsbeispiel so ausgelegt, dass eine elektrische Verbindung auch bei Rotation von Energiespeichereinheit und einer entsprechenden Steuereinheit relativ zueinander möglich ist.

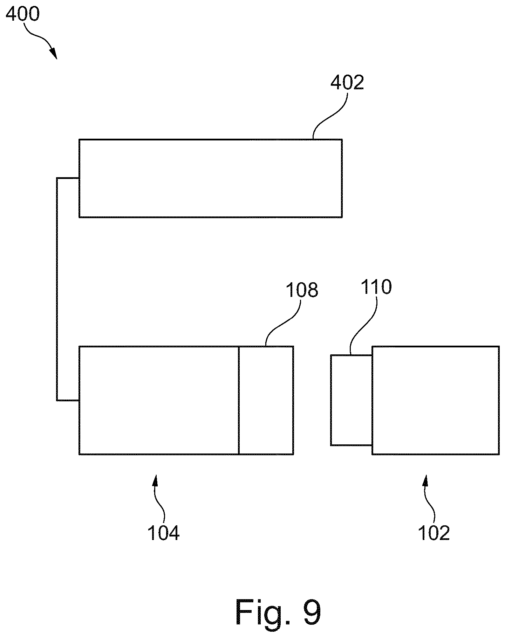

Zum Schluss soll noch die Verwendung der vorgestellten Steuereinrichtung als Teil einer Blutpumpeneinrichtung mit Bezug auf Fig. 9 beschrieben werden.

Fig. 9 zeigt eine Blutpumpeneinrichtung 400. Die Blutpumpeneinrichtung 400 umfasst die bereits beschriebene Steuereinheit 104 mit dem Rahmen 108 und die Energiespeichereinheit 102 mit dem Rahmeneinsteckelement 110. Die Steuereinheit 104 ist elektrisch mit einer Blutpumpe 402 verbunden, um die Blutpumpe 402 mit elektrischer Energie zu versorgen und mittels Steuersignalen zu steuern. Alternativ zu der Steuereinrichtung 100 kann jedoch auch jede andere der vorgestellten Steuereinrichtungen Teil der Blutpumpeneinrichtung sein.

Zusammenfassend beschreibt diese Offenbarung eine Steuereinrichtung (100) für eine Blutpumpe, umfassend eine Steuereinheit (104) und eine Energiespeichereinheit (102), die lösbar über eine Steckverbindung (106) elektrisch und mechanisch miteinander verbindbar sind. Dabei weist die Steckverbindung (106) zum Herstellen einer mechanischen Verbindung einen Rahmen (108) und ein in den Rahmen (108) entlang einer Einführungsrichtung (E) einführbares Rahmeneinsteckelement (110) auf, die durch ein Gehäuse (104G) der Steuereinheit (104) und ein Gehäuse (102G) der Energiespeichereinheit (102) ausgebildet sind. Ferner sind der Rahmen (108) und das Rahmeneinsteckelement (110) so ausgebildet, dass das Rahmeneinsteckelement (110) in zwei oder mehr Orientierungen, die sich durch eine relative Rotation zwischen Rahmen (108) und Rahmeneinsteckelement (110) um die Einführungsrichtung (E) voneinander unterscheiden, in den Rahmen (108) einführbar ist. Zudem weisen der Rahmen (108) und das Rahmeneinsteckelement (110) ein oder mehrere elektrische Kopplungspaare zum Herstellen einer elektrischen Verbindung zwischen Steuereinheit (104) und Energiespeichereinheit (102) auf und sind die einen oder mehreren elektrischen Kopplungspaare so angeordnet, dass eine elektrische Verbindung zwischen Energiespeichereinheit (102) und Steuereinheit (104) bei allen Orientierungen möglich ist.

## Patentansprüche

1. Steuereinrichtung (100) für eine Blutpumpe, umfassend eine Steuereinheit (104) und eine Energiespeichereinheit (102), die lösbar über eine Steckverbindung (106) elektrisch und mechanisch miteinander verbindbar sind, wobei
die Steckverbindung (106) zum Herstellen einer mechanischen Verbindung einen Rahmen (108) und ein in den Rahmen (108) entlang einer Einführungsrichtung (E) einführbares Rahmeneinsteckelement (110) aufweist, die durch ein Gehäuse (104G) der Steuereinheit (104) und ein Gehäuse (102G) der Energiespeichereinheit (102) ausgebildet sind,
der Rahmen (108) und das Rahmeneinsteckelement (110) so ausgebildet sind, dass das Rahmeneinsteckelement (110) in zwei oder mehr Orientierungen, die sich durch eine relative Rotation zwischen Rahmen (108) und Rahmeneinsteckelement (110) um die Einführungsrichtung (E) voneinander unterscheiden, in den Rahmen (108) einführbar ist, und
der Rahmen (108) und das Rahmeneinsteckelement (110) ein oder mehrere elektrische Kopplungspaare zum Herstellen einer elektrischen Verbindung zwischen Steuereinheit (104) und Energiespeichereinheit (102) aufweisen und die einen oder mehreren elektrischen Kopplungspaare so angeordnet sind, dass eine elektrische Verbindung zwischen Energiespeichereinheit (102) und Steuereinheit (104) bei allen Orientierungen möglich ist.

2. Steuereinrichtung (100) gemäß Anspruch 1, wobei der Rahmen (108) und das Rahmeneinsteckelement (110) in einer Ebene senkrecht zur Einführungsrichtung (E) ein oder mehr Spiegelachsen (A1, A2) aufweisen und die elektrischen Kopplungspaare symmetrische bzgl. der einen oder mehreren Spiegelachsen (A1, A2) angeordnet sind.

3. Steuereinrichtung (100) gemäß einem der Ansprüche 1 oder 2, wobei jedes Kopplungspaar einen an der Steuereinheit (104) angeordneten elektrischen Kontakt (104S) und eine an der Energiespeichereinheit (102) angeordnete elektrischen Gegenkontakt (102B) aufweist.

4. Steuereinrichtung (100) gemäß einem der vorhergehenden Ansprüche, wobei die Steckverbindung (106) ausgebildet ist, eine Vielzahl unterschiedlicher elektrischer Größen zwischen Steuereinheit (104) und Energiespeichereinheit (102) zu übertragen und die Steckverbindung (106) für die Übertragung jedes der unter-schiedlichen elektrischen Größen mindestens zwei elektrische Kopplungspaare umfasst.

5. Steuereinrichtung (100) gemäß einem der vorhergehenden Ansprüche, wobei die Steckverbindung (106) mindestens zwei elektrische Kopplungspaare aufweist, wobei die Kopplungspaare so ausgebildet sind, dass beim Einführen des Rahmeneinsteckelementes (110) in den Rahmen (108) eine elektrische Verbindung durch eines der beiden Kopplungspaare hergestellt wird, bevor eine elektrische Verbindung durch ein anderes Kopplungspaar hergestellt wird.

6. Steuereinrichtung (100) gemäß einem der vorhergehenden Ansprüche, wobei die Steckverbindung (106) einen lösbaren mechanischen Verschluss umfasst, der ausgebildet ist, nach dem Einführen des Rahmeneinsteckelementes (110) in den Rahmen (108) unabhängig von der Orientierung ein Entfernen des Rahmeneinsteckelementes (110) aus dem Rahmen (108) zu verhindern.

7. Steuereinrichtung (100) gemäß einem der vorhergehenden Ansprüche, wo-bei
ein elektrisches Kopplungspaar als elektrischen Kontakt eine Buchse (102B) und als elektrischen Gegenkontakt einen in die Buchse (102B) einführbaren Stift (104S) umfasst, wobei der Stift (104S) in Einführungsrichtung (E) längenveränderbar ist und eine Feder (104.S.3) umfasst, die ausgebildet ist, mittels einer Rückstellkraft einem Zusammendrücken des Stiftes (104S) entgegenzuwirken, und
Buchse (102B) und Stift (104S) so angeordnet sind, dass im verbundenen Zustand der Steckverbindung (106) der Stift (104S) durch die Buchse (102B) zusammengedrückt wird.

8. Steuereinrichtung (100) gemäß Anspruch 6 und Anspruch 7, wobei die in dem elektrischen Kopplungspaar verwendete Feder (104S.3) so ausgebildet ist, dass die Feder (104S.3) eine Rückstellkraft ausbilden kann, die größer ist als eine zum Verschließen des mechanischen Verschlusses benötigte Kraft.

9. Steuereinrichtung (100) gemäß einem der vorhergehenden Ansprüche, wobei die Steckverbindung (106) ein Dichtungselement (112) umfasst, das so angeordnet ist, dass in einem verbundenen Zustand der Steckverbindung (106) ein In-Kontakt-kommen von Staub und/oder Wasser mit dem einen oder den mehreren elektrischen Kopplungspaaren reduziert wird.

10. Steuereinrichtung (200) gemäß einem der vorhergehenden Ansprüche, wo-bei ein elektrisches Kopplungspaar als elektrischen Kontakt eine Stabelektrode (104S) und als elektrischen Gegenkontakt eine Stabbuchse (102B) umfasst, wobei
die Stabelektrode (104S) entlang einer axialen Richtung der Stabelektrode (104S) eine Vielzahl von Stabkontaktelementen (104S.1-5) zur unabhängigen Übertragung von zwei oder mehr elektrischen Größen aufweist, und
die Stabbuchse (102B) entsprechend der Stabelektrode (102B) entlang einer axialen Richtung der Stabbuchse (102B) eine Vielzahl von Buchsenkontaktelementen (102B.1-5) auf-weist.

11. Steuereinrichtung (100) gemäß einem der vorhergehenden Ansprüche, wobei ein elektronisches Kopplungspaar einen elektrischen Abnehmer und eine elektrische Kontaktfläche aufweist, wobei
die elektrische Kontaktfläche an einer senkrecht zur Einführungsrichtung (E) zeigenden Seite des Rahmeneinsteckelementes (110) angeordnet ist, und
der elektrische Abnehmer entsprechend zur elektrischen Kontaktfläche an einer Innenseite des Rahmens (108) angeordnet ist.

12. Steuereinrichtung (100) gemäß einem der vorhergehenden Ansprüche, wobei der Rahmen (108) und das Rahmeneinsteckelement (110) in einer Ebene senkrecht zur Einführungsrichtung (E) eine Grundform aufweisen, die so ausgebildet ist, dass das Rahmeneinsteckelement (110) in zwei Orientierungen, die sich durch eine relative Rotation zwischen Rahmen (108) und Rahmeneinsteckelement (110) von 180° um eine Einführungsrichtung (E) voneinander unterscheiden, in den Rahmen (108) einführbar ist.

13. Steuereinrichtung (300) gemäß einem der vorhergehenden Ansprüche, wobei Kontakte (304B.1-5) der Kopplungspaare des Rahmens (308) und des Rahmeneinsteckelementes (310) cluster-artig innerhalb des Rahmens (308) bzw. am Rahmeneinsteckelement (310) angeordnet sind, wobei ein Abstand zwischen Kontakten eines Clusters (C1, C2) kleiner ist als ein Abstand zwischen den Clustern (C1, C2).

14. Blutpumpeneinrichtung (400), umfassend:
eine implantierbare Blutpumpe (402), und
eine Steuereinrichtung (100) gemäß einem der vorhergehenden Ansprüche, wobei die Steuereinrichtung (100) elektrisch mit der Blutpumpe (402) verbunden ist.

## Claims

1. A control device (100) for a blood pump, comprising a control unit (104) and an energy storage unit (102) which are detachably connectable to one another electrically and mechanically via a plug connection (106), wherein
the plug connection (106) for establishing a mechanical connection has a frame (108) and a frame insertion element (110) insertable into the frame (108) along an insertion direction (E), said frame and frame insertion element being formed by a housing (104G) of the control unit (104) and a housing (102G) of the energy storage unit (102),
the frame (108) and the frame insertion element (110) are configured such that the frame insertion element (110) is insertable into the frame (108) in two or more orientations which differ from one another by a relative rotation between the frame (108) and the frame insertion element (110) about the insertion direction (E), and
the frame (108) and the frame insertion element (110) have one or more electrical coupling pairs for establishing an electrical connection between the control unit (104) and the energy storage unit (102), and the one or more electrical coupling pairs are arranged such that an electrical connection between the energy storage unit (102) and the control unit (104) is possible in all orientations.

2. The control device (100) according to claim 1, wherein the frame (108) and the frame insertion element (110) have one or more mirror axes (A1, A2) in a plane perpendicular to the insertion direction (E) and the electrical coupling pairs are arranged symmetrically with respect to the one or more mirror axes (A1, A2).

3. The control device (100) according to any one of claims 1 or 2, wherein each coupling pair comprises an electrical contact (104S) arranged on the control unit (104) and a mating electrical contact (102B) arranged on the energy storage unit (102).

4. The control device (100) according to any one of the preceding claims, wherein the plug connection (106) is configured to transmit a plurality of different electrical quantities between control unit (104) and energy storage unit (102) and the plug connection (106) comprises at least two electrical coupling pairs for the transmission of each of the different electrical quantities.

5. The control device (100) according to any one of the preceding claims, wherein the plug connection (106) has at least two electrical coupling pairs, wherein the coupling pairs are configured such that, when the frame insertion element (110) is inserted into the frame (108), an electrical connection is established by one of the two coupling pairs before an electrical connection is established by another coupling pair.

6. The control device (100) according to any one of the preceding claims, wherein the plug connection (106) comprises a releasable mechanical lock which is configured to prevent removal of the frame insertion element (110) from the frame (108) after insertion of the frame insertion element (110) into the frame (108), regardless of the orientation.

7. The control device (100) according to any one of the preceding claims, wherein
an electrical coupling pair comprises a socket (102B) as electrical contact and a pin (104S) insertable into the socket (102B) as electrical mating contact, wherein the pin (104S) is variable in length in the insertion direction (E) and comprises a spring (104.S.3) which is configured to counteract a compression of the pin (104S) by means of a restoring force, and
the socket (102B) and pin (104S) are arranged such that the pin (104S) is compressed by the socket (102B) when the plug connection (106) is in the connected state.

8. The control device (100) according to claim 6 and claim 7, wherein the spring (104S.3) used in the electrical coupling pair is configured such that the spring (104S.3) may form a restoring force that is greater than a force required to close the mechanical lock.

9. The control device (100) according to any one of the preceding claims, wherein the plug connection (106) comprises a sealing element (112) which is arranged such that, in a connected state of the plug connection (106), the extent to which dust and/or water come into contact with the one or more electrical coupling pairs is reduced.

10. The control device (200) according to any one of the preceding claims, wherein an electrical coupling pair comprises a rod electrode (104S) as electrical contact and a rod socket (102B) as electrical mating contact, wherein
the rod electrode (104S) has a plurality of rod contact elements (104S.1-5) along an axial direction of the rod electrode (104S) for independently transmitting two or more electrical quantities, and
the rod socket (102B) has a plurality of socket contact elements (102B.1-5) along an axial direction of the rod socket (102B) corresponding to the rod electrode (102B).

11. The control device (100) according to any one of the preceding claims, wherein an electronic coupling pair comprises an electrical collector and an electrical contact surface, wherein
the electrical contact surface is arranged on a side of the frame insertion element (110) pointing perpendicularly to the insertion direction (E), and
the electrical collector is arranged on an inner side of the frame (108) corresponding to the electrical contact surface.

12. The control device (100) according to any one of the preceding claims, wherein the frame (108) and the frame insertion element (110) have a basic shape in a plane perpendicular to the insertion direction (E), said basic shape being configured such that the frame insertion element (110) is insertable into the frame (108) in two orientations, which differ from each other by a relative rotation between frame (108) and frame insertion element (110) of 180° about an insertion direction (E).

13. The control device (300) according to any one of the preceding claims, wherein contacts (304B.1-5) of the coupling pairs of the frame (308) and the frame insertion element (310) are arranged in a cluster-like manner within the frame (308) or on the frame insertion element (310), wherein a distance between contacts of a cluster (C1, C2) is smaller than a distance between the clusters (C1, C2).

14. A blood pump device (400) comprising:
an implantable blood pump (402) and
a control device (100) according to any one of the preceding claims, wherein the control device (100) is electrically connected to the blood pump (402).

## Revendications

1. Dispositif de commande (100) pour une pompe à sang, comprenant une unité de commande (104) et une unité de stockage d'énergie (102) qui peuvent être connectées l'une à l'autre électriquement et mécaniquement de manière amovible par l'intermédiaire d'une connexion enfichable (106), dans lequel
la connexion enfichable (106) comprend, en vue de l'établissement d'une liaison mécanique, un cadre (108) et un élément formant fiche de cadre (110) pouvant être inséré dans le cadre (108) dans une direction d'insertion (E), lesquels sont formés par un boîtier (104G) de l'unité de commande (104) et un boîtier (102G) de l'unité de stockage d'énergie (102),
le cadre (108) et l'élément formant fiche de cadre (110) sont réalisés de sorte que l'élément formant fiche de cadre (110) peut être inséré dans le cadre (108) selon deux ou plus de deux orientations qui diffèrent l'une de l'autre par la rotation relative entre le cadre (108) et l'élément formant fiche de cadre (110) autour de la direction d'insertion (E), et
le cadre (108) et l'élément formant fiche de cadre (110) présentent une ou plusieurs paire(s) de couplage électrique afin d'établir une connexion électrique entre l'unité de commande (104) et l'unité de stockage d'énergie (102), et la ou les paire(s) de couplage électrique est/sont agencée(s) de sorte qu'une connexion électrique entre l'unité de stockage d'énergie (102) et l'unité de commande (104) est possible dans toutes les orientations.

2. Dispositif de commande (100) selon la revendication 1, dans lequel le cadre (108) et l'élément formant fiche de cadre (110) présentent un ou plusieurs axe(s) de symétrie (A1, A2) dans un plan perpendiculaire à la direction d'insertion (E) et les paires de couplage électrique sont agencées symétriquement par rapport au ou aux axe(s) de symétrie (A1, A2).

3. Dispositif de commande (100) selon la revendication 1 ou 2, dans lequel chaque paire de couplage présente un contact électrique (1045) agencé au niveau de l'unité de commande (104) et un contre-contact électrique (102B) agencé au niveau de l'unité de stockage d'énergie (102).

4. Dispositif de commande (100) selon l'une quelconque des revendications précédentes, dans lequel la connexion enfichable (106) est conçue pour transmettre une pluralité de variables électriques différentes entre l'unité de commande (104) et l'unité de stockage d'énergie (102), et la connexion enfichable (106) comprend au moins deux paires de couplage électrique afin de transmettre chacune des variables électriques différentes.

5. Dispositif de commande (100) selon l'une quelconque des revendications précédentes, dans lequel la connexion enfichable (106) présente au moins deux paires de couplage électrique, dans lequel les paires de couplage sont réalisées de sorte que, lorsque l'élément enfichable de cadre (110) est inséré dans le cadre (108), une connexion électrique est établie grâce à l'une des deux paires de couplage avant qu'une connexion électrique ne soit établie grâce à une autre paire de couplage.

6. Dispositif de commande (100) selon l'une quelconque des revendications précédentes, dans lequel la connexion enfichable (106) comprend une fermeture mécanique libérable qui est conçue pour empêcher le retrait de l'élément formant fiche de cadre (110) hors du cadre (108) après l'insertion de l'élément formant fiche de cadre (110) dans le cadre (108), indépendamment de l'orientation.

7. Dispositif de commande (100) selon l'une quelconque des revendications précédentes, dans lequel
une paire de couplage électrique comprend une douille (102B) faisant office de contact électrique et une broche (104S) insérable dans la douille (102B) et faisant office de contre-contact électrique, dans lequel la broche (104S) est de longueur variable dans la direction d'insertion (E) et comprend un ressort (104.5.3) conçu pour contrer une compression de la broche (104S) au moyen d'une force de rappel, et
la douille (102B) et la broche (104S) sont agencées de sorte que, lorsque la connexion enfichable (106) se trouve dans un état de connexion, la broche (104S) est compressée par la douille (102B).

8. Dispositif de commande (100) selon la revendication 6 et la revendication 7, dans lequel le ressort (104S.3) utilisé dans la paire de couplage électrique est réalisé de sorte que le ressort (104S.3) peut former une force de rappel qui est supérieure à une force requise pour fermer la fermeture mécanique.

9. Dispositif de commande (100) selon l'une quelconque des revendications précédentes, dans lequel la connexion enfichable (106) comprend un élément d'étanchéité (112) agencé de sorte que, dans un état connecté de la connexion enfichable (106), une mise en contact de poussière et/ou d'eau avec la ou les paire(s) de couplage électrique est réduite.

10. Dispositif de commande (200) selon l'une quelconque des revendications précédentes, dans lequel une paire de couplage électrique comprend une électrode à tige (104S) faisant office de contact électrique et une douille à tige (102B) faisant office de contre-contact électrique, dans lequel
l'électrode à tige (104S) présente une pluralité d'éléments de contact à tige (104S.1 à 5) le long d'une direction axiale de l'électrode à tige (104S) en vue de la transmission indépendante de deux ou plus de deux variables électriques, et
la douille de tige (102B) présente, d'une manière qui correspond à l'électrode de tige (102B), une pluralité d'éléments de contact de douille (102B.1 à 5) le long d'une direction axiale de la douille à tige (102B).

11. Dispositif de commande (100) selon l'une quelconque des revendications précédentes, dans lequel une paire de couplage électronique présente un capteur électrique et une surface de contact électrique, dans lequel
la surface de contact électrique est agencée sur un côté de l'élément formant fiche de cadre (110) regardant perpendiculairement à la direction d'insertion (E), et
le capteur électrique est agencé sur un côté intérieur du cadre (108) d'une manière qui correspond à la surface de contact électrique.

12. Dispositif de commande (100) selon l'une quelconque des revendications précédentes, dans lequel le cadre (108) et l'élément formant fiche de cadre (110) présentent, dans un plan perpendiculaire à la direction d'insertion (E), une forme de base qui est conçue de sorte que l'élément formant fiche de cadre (110) peut être inséré dans le cadre (108) selon deux orientations qui diffèrent l'une de l'autre par la rotation relative de 180° entre le cadre (108) et l'élément formant fiche de cadre (110) autour d'une direction d'insertion (E).

13. Dispositif de commande (300) selon l'une quelconque des revendications précédentes, dans lequel les contacts (304B.1 à 5) des paires de couplage du cadre (308) et de l'élément formant fiche de cadre (310) sont agencés en grappe à l'intérieur du cadre (308) ou au niveau de l'élément formant fiche de cadre (310), dans lequel une distance entre les contacts d'une grappe (C1, C2) est inférieure à une distance entre les grappes (C1, C2).

14. Dispositif de pompe à sang (400), comprenant :
une pompe à sang (402) pouvant être implantée, et
un dispositif de commande (100) selon l'une quelconque des revendications précédentes, dans lequel le dispositif de commande (100) est connecté électriquement à la pompe à sang (402).
